# EUROPÄISCHE PATENTSCHRIFT

(11) **EP 0 559 929 B1**
(45) Veröffentlichungstag und Bekanntmachung des Hinweises auf die Patenterteilung: **26.06.1996**
(21) Anmeldenummer: 92104030.9
(22) Anmeldetag: 09.03.1992
(51) Int. Cl.: G01N 33/00

(54) **Anordnung zum Entfernen von Störkomponenten aus Messgasen für die Gasanalyse**
Device for the elimination of unwanted components from gases for gas analyses
Dispositif pour l'élimination de composantes parasites dans des gaz pour l'analyse de gaz

(43) Veröffentlichungstag der Anmeldung: 15.09.1993
(73) Patentinhaber: SIEMENS AKTIENGESELLSCHAFT, D-80333 München (DE)
(72) Erfinder: Schroth, Ingeborg, W-7517 Waldbronn (DE)

(56) Entgegenhaltungen:
- EP-A- 0 362 889
- DE-U- 9 107 975
- US-A- 3 897 679
- US-A- 4 030 887
- PATENT ABSTRACTS OF JAPAN, Band 10, Nr. 282(P-500), 25. September 1986/

## Beschreibung

In dem Buch "Messen, Steuern, Regeln in der chemischen Technik", Band II, Springer-Verlag 1980, Seiten 37 bis 39 und Seiten 45 bis 47 sind Infrarot-Gasanalysatoren beschrieben, mit denen die Differenz der Absorption von Infrarot-Strahlung durch die Meßkomponente eines Meßgases und durch ein Vergleichsgas gemessen und daraus die Konzentration der Meßkomponente im Meßgas ermittelt wird. Die Meßgenauigkeit derartiger IR-Analysatoren kann beeinträchtigt werden, wenn das Meßgas eine Störkomponente mit IR-Absorptionsbanden enthält, die mit den IR-Banden des Meßgases überlappen. Zum Beseitigen dieser Meßwertverfälschung ist neben verschiedenen anderen Maßnahmen in dem genannten Buch auf Seite 612/613 angegeben, Störkomponenten durch Absorption mittels geeigneter Chemikalien zu entfernen. Dadurch entsteht die Gefahr der zusätzlichen Meßgasverfälschung durch unerwünschte Reaktionen mit dem Meßgas. Je nach Beschaffenheit der benutzten Chemikalie werden unterschiedliche Absorptionsgefäße verwendet, z. B. Absorptionsrohre oder Patronen für feste Chemikalien. Die Chemikalien werden während des Meßvorganges verbraucht und müssen daher von Zeit zu Zeit ersetzt werden.

Der vorliegenden Erfindung liegt die Aufgabe zugrunde, aus einem Meßgas mit einem einwertigen Alkohol, insbesondere Äthanol als Meßkomponente die Störkomponente Cineol, insbesondere 1,8-Cineol zu entfernen.

Erfindungsgemäß wird diese Aufgabe mit den in den kennzeichnenden Teilen der Ansprüche 1 und 2 angegebenen Maßnahmen gelöst.

Die Erfindung beruht auf der Erkenntnis, daß Silikongummi aus einem Gemisch aus Äthanol und 1,8-Cineol selektiv das Cineol adsorbiert. Es wird keine Chemikalie eingesetzt, die verbraucht und erneuert werden muß, sondern ein Adsorber aus Silikongummi, aus dem, zweckmäßig nach jedem Meßvorgang, die adsorbierte Störkomponente ausgespült wird. Dadurch wird vermieden, daß der Adsorber mit der Störkomponente gesättigt wird und seine Eigenschaft als Adsorber verliert. Selbstverständlich darf während eines Meßvorganges nur so viel Meßgas über den Adsorber strömen, daß dieser auch nicht annähernd mit der Störkomponente gesättigt wird und seine Funktion auch am Ende des Meßvorganges noch erfüllt.

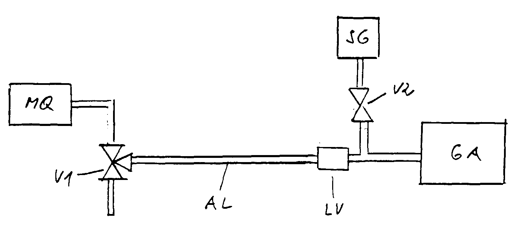

Anhand der Zeichnung werden im folgenden die Erfindung sowie weitere Ausgestaltungen und Ergänzungen näher beschrieben und erläutert.

In der Zeichnung ist mit MQ eine Meßgasquelle bezeichnet, an die mit einem Ventil V1 über eine Adsorberleitung AL und einen Leitungsverbinder LV ein nicht dispersiver IR-Gasanalysator GA anschließbar ist. Mit diesem soll die Konzentration von Äthanol im Meßgas gemessen werden. Die Infrarot-Absorptionsbanden von Äthanol sind mit den Absorptionsbanden von 1,8-Cineol überlagert, das im Meßgas als Störkomponente auftreten kann. Ohne zusätzliche Maßnahmen kann daher der Meßwert für die Äthanol-Konzentration verfälscht werden. Zur Vermeidung solcher Meßfehler ist im Strömungsweg des Meßgases ein Adsorber in Form der Adsorptionsleitung AL angeordnet. Diese besteht aus Silikongummi bzw. weist sie auf ihrer Innenseite Teile aus Silikongummi auf oder sie ist innen damit beschichtet. Silikongummi hat die Eigenschaft, Cineol zu adsorbieren, nicht aber oder in nur unwesentlichem Maße Äthanol. Das dem Gasanalysator GA zugeführte Meßgas ist daher von der Störkomponente Cineol befreit. Die Adsorptionsleitung AL ist hinsichtlich Länge und Querschnitt so bemessen, daß das Meßgas sich genügend lange darin aufhält und mit dem Silikongummi ausreichend in Kontakt ist. Auch soll die Oberfläche des Silikongummis ausreichen groß sein. Zweckmäßig besteht die Adsorberleitung aus einem Schlauch aus Silikongummi mit rauher innerer Oberfläche.

Der Innenraum der Adsorberleitung darf das Leitungsvolumen zwischen der Meßgasquelle und dem Gasanalysator nicht wesentlich erhöhen, d. h., es muß wesentlich kleiner sein, als das für einen Meßvorgang zur Verfügung stehende Meßgasvolumen.

Nach einem Meßvorgang wird das Dreiwegeventil V1 umgeschaltet und ein Ventil V2 geöffnet, so daß aus einer Spülgasquelle SG Spülgas durch die Adsorberleitung AL strömt, das über das Ventil V1 ins Freie gelangt. Als Spülgas kann Luft verwendet werden. Besonders geeignet ist selbstverständlich ein Spülgas, das besonders leicht die Störkomponente löst oder absorbiert, ohne selbst von Silikongummi adsorbiert zu werden. Die Strömungsrichtung des Spülgases ist der des Meßgases entgegengesetzt, so daß die Störkomponente, die sich vorwiegend auf der linken Seite der Adsorberleitung AL abgelagert hat, sich nicht über die Adsorberleitung AL verteilen kann. Das Spülgas wird ferner dem Gasanalysator GA zugeführt, damit alle Teile, die mit Meßgas in Berührung kommen, bespült werden. Nach dem Spülvorgang wird das Ventil V2 geschlossen und das Ventil V1 umgeschaltet.

Im dargestellten Ausführungsbeispiel sind der Meßgas- und der Spülgasstrom entgegengesetzt gerichtet. Es ist aber auch möglich, daß sie gleichgerichtet sind. In diesem Falle schaltet das Dreiwegeventil V1 während des Spülvorgangs eine Spülgasquelle an die Adsorberleitung AL. Das nicht durch den Gasanalysator GA strömende Spülgas gelangt über das während des Spülvorganges geöffnete Ventil V2 ins Freie. Das Ventil V1 erübrigt sich, wenn die Meßgasquelle während des Spülvorganges von der Leitung AL getrennt wird, was zwangsläufig dann der Fall ist, wenn an Meßgas aus unterschiedlichen Meßgasquellen gemessen wird.

Eine Messung kann erst dann durchgeführt werden, wenn das gesamte Spülgas vom einströmenden Meßgas aus den Leitungen gedrückt ist. Daraus ergibt sich, daß nicht nur das Volumen der Adsorberleitung an das Meßgasvolumen eines Meßvorganges angepaßt sein muß, sondern daß auch das Spülgasvolumen auf das Volumen der Adsorberleitung und damit auf das Meßgasvolumen abgestimmt ist. Je größer das Meßgasvolumen ist, um so größer kann das Volumen der Adsorberleitung sein und es muß auch größer sein, damit auch mehr Adsorberfläche zur Verfügung steht. Anstelle einer Adsorberleitung oder Schlauches kann in die Meßgasleitung auch ein Behälter eingesetzt sein, der einen Block aus porösem Silikongummi enthält, durch den das Meß- bzw. das Spülgas strömt. Zur Verringerung des Strömungswiderstandes kann der Block mit Waben oder schlitzförmigen Durchlässen versehen sein.

## Patentansprüche

1. Verfahren zum Ausscheiden von Störkomponenten aus Meßgasen für die Gasanalyse, **dadurch gekennzeichnet**, daß zum Messen der Konzentration von einwertigem Alkohol, insbesondere Äthanol, in einem Cineol als Störkomponente enthaltenden Meßgas zyklisch abwechselnd das Meßgas und ein Spülgas über Silikongummi geleitet werden.

2. Anordnung zur Durchführung des Verfahrens nach Anspruch 1, **gekennzeichnet durch** einen Adsorber (AL) aus Silikongummi, der zyklisch in den Strömungsweg des Meßgases und den von Spülgas schaltbar ist.

3. Anordnung nach Anspruch 2, **dadurch gekennzeichnet,** daß der Adsorber aus einer Leitung (AL) mit einer Innenbeschichtung aus Silikongummi besteht.

4. Anordnung nach Anspruch 2, **dadurch gekennzeichnet,** daß der Adsorber ein Schlauch aus Silikongummi ist.

5. Anordnung nach Anspruch 1 oder 2, **dadurch gekennzeichnet,** daß in einem weitgehend inerten Schlauch zusätzlich Silikongummiflächen eingebracht sind.

6. Anordnung nach einem der Ansprüche 2 bis 5, **dadurch gekennzeichnet,** daß das Meß- und das Spülgasvolumen groß im Vergleich zum Gasvolumen des Adsorbers sind.

7. Anordnung nach einem der Ansprüche 2 bis 6, **dadurch gekennzeichnet,** daß die Richtungen der Meß- und Spülgasströme im Adsorber (AL) entgegengesetzt sind.

8. Anordnung nach einem der Ansprüche 2 bis 7, **dadurch gekennzeichnet,** daß der gleiche Gasweg für Meß- und Spülvorgang zeitlich nacheinander benutzt wird.

## Claims

1. Process for separating disturbance constituents from measuring gases for gas analysis, characterised in that, for measuring the concentration of monohydric alcohol, in particular ethanol, in a measuring gas containing cineole as a disturbance constituent, the measuring gas and a scavenging gas are passed over silicon rubber in alternating cycles.

2. Arrangement for carrying out the process according to claim 1, characterised by an adsorbent (AL) made of silicon rubber which can be connected cyclically to the flow path of the measuring gas and to the flow path of the scavenging gas.

3. Arrangement according to claim 2, characterised in that the adsorbent consists of a pipe (AL) having an inner coating of silicon rubber.

4. Arrangement according to claim 2, characterised in that the adsorbent is a hose made of silicon rubber.

5. Arrangement according to claim 1 or 2, characterised in that silicon rubber surfaces are additionally placed in a substantially inert hose.

6. Arrangement according to one of claims 2 to 5, characterised in that the measuring gas volume and the scavenging gas volume are large in comparison to the gas volume of the adsorbent.

7. Arrangement according to one of claims 2 to 6, characterised in that the directions of flow of the measuring gas and the scavenging gas in the adsorbent (AL) are opposed.

8. Arrangement according to one of claims 2 to 7, characterised in that the same gas path is used for the measuring process and for the rinsing process successively in time.

## Revendications

1. Procédé de séparation de constituants de gaz à mesurer gênants pour l'analyse des gaz, caractérisé en ce qu'il consiste, pour mesurer la concentration d'un alcool monovalent, notamment de l'éthanol, d'un gaz à mesurer contenant du cinéol comme constituant gênant, à envoyer cycliquement en alternance le gaz à mesurer et un gaz de balayage sur du caoutchouc de silicone.

2. Dispositif pour la mise en oeuvre du procédé suivant la revendication 1, caractérisé par un adsorbeur (AL) en caoutchouc de silicone, qui peut être branché cycliquement dans le trajet d'écoulement du gaz de mesure et dans celui du gaz de balayage.

3. Dispositif suivant la revendication 2, caractérisé en ce que l'adsorbeur est constitué d'un conduit (AL) ayant un revêtement intérieur en caoutchouc de silicone.

4. Dispositif suivant la revendication 2, caractérisé en ce que l'adsorbeur est un tuyau souple en caoutchouc de silicone.

5. Dispositif suivant la revendication 1 ou 2, caractérisé en ce que des surfaces de caoutchouc de silicone supplémentaires sont placées dans un tuyau souple sensiblement inerte.

6. Dispositif suivant l'une des revendications 2 à 5, caractérisé en ce que le volume du gaz de mesure et du gaz de balayage est grand par rapport au volume de gaz de l'adsorbeur.

7. Dispositif suivant l'une des revendications 2 à 6, caractérisé en ce que les sens des courants du gaz de mesure et du gaz de balayage dans l'adsorbeur (AL) sont opposés.

8. Dispositif suivant l'une des revendications 2 à 7, caractérisé en ce que le même trajet des gaz est utilisé successivement dans le temps pour l'opération de mesure et pour l'opération de balayage.
